**Europäisches Patentamt**

**European Patent Office**

**Office européen des brevets**

(19)

(11) Publication number: **0 115 627**
**B1**

# (12) EUROPEAN PATENT SPECIFICATION

(45) Date of publication of patent specification: **01.04.87**

(21) Application number: **83113070.3**

(22) Date of filing: **23.12.83**

(51) Int. Cl.⁴: **A 61 K 37/02**

(54) **Enhancement of intranasal absorption of calcitonin by formulation with surfactants.**

(30) Priority: **29.12.82 US 454128**

(43) Date of publication of application:
**15.08.84 Bulletin 84/33**

(45) Publication of the grant of the patent:
**01.04.87 Bulletin 87/14**

(84) Designated Contracting States:
**AT BE CH DE FR GB IT LI LU NL SE**

(56) References cited:
**DE-A-2 254 061**
**GB-A-1 527 605**
**GB-A-1 548 984**
**US-A-4 241 051**

(73) Proprietor: **Armour Pharmaceutical Company**
**303 South Broadway**
**Tarrytown New York 10591 (US)**

(72) Inventor: **Mufson, Daniel**
**24 Hillside Drive**
**Thiells, N.Y. (US)**
Inventor: **Hanson, Musetta A.**
**1 Consulate Drive**
**Tuckhoe, N.Y. (US)**

(74) Representative: **Patentanwälte Grünecker, Dr.**
**Kinkeldey, Dr. Stockmair, Dr. Schumann, Jakob,**
**Dr. Bezold, Meister, Hilgers, Dr. Meyer-Plath**
**Maximilianstrasse 58**
**D-8000 München 22 (DE)**

The file contains technical information
submitted after the application was filed and
not included in this specification

Courier Press, Leamington Spa, England.

## Description

The present invention relates to a pharmaceutical composition for the treatment of disorders of bone metabolism which comprises an aqueous or non-aqueous medium suitable for intranasal administration containing a therapeutically effective amount of calcitonin.

Calcitonin is a polypeptide hormone isolated from different organs in different species, including man and salmon, or obtained via synthetic routes. Calcitonin is recognized as being effective in diminishing hypercalcemia and decreasing plasma phosphate concentrations in patients with hyperparathyroidism, idiophatic hypercalcemia of infancy, vitamin D intoxication, and osteolytic bone metastases. While direct renal effects and actions on the gastrointestinal tract are recognized, calcitonin is best known for its effect on bone. Its use has proved to be effective in diseases characterized by increased skeletal resorption and abnormal bone formation, such as occurs for example, in Paget's disease.

The method of administration of calcitonin is predominantly by injection, although efforts were made in the prior art to use other modes of administration, especially for the treatment of localized conditions. While injectable administration by physicians of calcitonin is proper for short-term therapy, administration of calcitonin by injection to patients in need of long-term calcitonin therapy has a serious problem. Not only is it costly to patients to have physicians do the administration of calcitonin for extended periods of time but it is also painful and inconvenient. Nor can calcitonin be given orally to patients as it will be destroyed by the digestive juices in the gastrointestinal tract.

US—A—4 241 051 discloses a method for the treatment of pathological processes in bone and connective tissue, which comprises the topical application of calcitonin. Preferred compositions for use in the method are ear drops comprising an aprotic solvent or an aqueous medium containing a surface active agent, which may be cationic, anionic or nonionic.

In view of the foregoing, it is apparent that a serious need exists for a different route of delivery of calcitonin to patients suffering from conditions that require prolonged calcitonin therapy.

Nasal preparations are known in the prior art. Generally, nasal preparations comprise an oil-in-water or water-in-oil emulsion or an oily solvent base suitable for use on the mucous membranes, such as mineral or vegetable oils and fatty acid esters and one or more chemicals which are soluble in the base. Such preparations usually contain one or more active drugs intended to alleviate or mitigate a condition in the body by their adsorption into the blood stream through the mucous membrane of the nose.

While small molecules such as propranolol are efficiently absorbed intranasally, large molecules such as calcitonin show little if any absorption. The purpose of this invention is to find agents capable of increasing the bioavailability of calcitonin, so that the cost of therapy is reasonable. From "Diabetes", volume 27, pages 296 to 299 (1978), it is known that the nasal absorption of certain drugs may be facilitated by the use of surfactants in such nasal preparation. For example, insulin and polypeptides were found to have an improved absorption rate when used in a solution containing a surfactant.

GB—A—15 27 605 discloses an aqueous insulin preparation for intranasal administration having a pH value over 4.7, which comprises water, from 0.1 to 10 weight % of insulin and from 0.1 to 20 weight % of a substance having surface activity, which is a nonionic surface-active agent, an amphoteric surface-active agent, an anionic surface-active agent, surfactin, a bile acid salt or a saponin.

It is the object of the present invention to provide a pharmaceutical composition comprising calcitonin, which is suitable for intranasal administration. This object is achieved by a pharmaceutical composition for the treatment of disorders of bone metabolism, which comprises an aqueous or non-aqueous medium suitable for intranasal administration containing a therapeutically effective amount of calcitonin, a bile acid salt surface active agent, and a buffer.

It has been found that hypercalcemia, Paget's disease and other disorders of bone metabolism can be advantageously treated by intranasal application of calcitonin contained in a nasal preparation having an absorption promoter, which is a bile acid salt, and a buffer as essential ingredients. Such preparations possess enhanced absorption across the nasal mucosa when applied intranasally, but cause no irritation or discomfort on extended use.

By means of the compositions according to the invention, calcitonin may be intranasally administered to a mammal via a novel dosage form, such as a solution, ointment, or gel.

Calcitonin is a peptide hormone of 32 amino acids with a disulfide bond at 1—7 in the amino terminus of the molecule. These first seven amino acids with the disulfide bond seem essential for activity and this sequence is preserved from species to species.

The amount of calcitonin contained in the preparation of the present invention may vary according to various parameters, such as the nature of the preparation, the particular kind or activity of calcitonin employed and the condition or ailment to be treated with the preparations. In general, the concentrations are somewhat higher than those found in compositions for the systemic administration of calcitonin. It has been found that a concentration level of 1 to 150 micrograms per ml and preferably 2 to 30 micrograms per ml achieve the desired result. The levels of administration of calcitonin also vary somewhat from those used systemically. In the case of human patients, for example, amounts of from 0.7 to 70 micrograms, particularly from 1 to 25 micrograms, are usually appropriate for single dosages given and repeated as often as the physician finds it necessary and such dosages correspond generally to about 0.01 to 1 micrograms, and particularly 0.03 to 0.35 micrograms, per kilogram of body weight. (The above concentration and dosage levels of calcitonin apply to calcitonin with a potency of about 4000 International Units per mg and may be adjusted pro rata for calcitonin of other potencies.)

The diluent base or vehicle used in accordance with the present invention may be non-aqueous or aqueous. In the former case the diluents are physiologically acceptable polar solvents. Preferred solvents of this type are those with which it is possible to make a solution of adequate concentration of dissolved calcitonin. Examples of these solvents include dimethyl sulphoxide, dimethylformamide, dimethyllauramide, polyhydroxy alcohols, vegetable and mineral oils. If desired, such non-aqueous media may be mixed with water to form the diluent of the preparation.

However, the degree of physiological acceptability of the non-aqueous diluents is generally less than that of aqueous media and the preferred diluent is therefore water without the addition of organic solvents.

In the preparations of the present invention, calcitonin is used in combination with a bile acid salt surface active agent as absorption promoter. The amount of such an agent may be in the range from 0.01 to 10% w/v and preferably 0.05 to 1.0% w/v, the amount depending on the specific surfactant used. The amount is generally kept as low as possible since above a certain level no further enhancement of absorption can be achieved and also too high of a surfactant level may cause irritation of the nasal mucosa. Such bile acid salt surface active agents may be, for example, sodium taurocholate, sodium cholate, sodium deoxycholate or sodium glycocholate.

The preparations of the present invention preferably contain a phosphate or acetate buffer in the range of 0.01 M to 0.5 M and preferably in the range of 0.05 M to 0.2 M. This concentration was found effective to provide stability of the dissolved calcitonin in the diluent base or vehicle.

The preparations of the present invention may also contain other additives, such antioxidants, stabilizers, tonicity adjusters, viscosity builders or preservatives.

The concentration of these additives may vary according to the particular additive used and the desired result sought. In general, the concentrations for these additives will be in the range as follows:

| Additives | % W/V |
|---|---|
| Antioxidants | 0.01—0.2 |
| Stabilizers | 0.01—2.0 |
| Tonicity adjuster | 0.01—0.5 |
| Viscosity builders | 0.1—2.0 |
| Preservatives | 0.001—2.0 |

While the use of the kind and concentration of additives will be well within the ability of the skilled artisan, the following will serve as illustration for two additives generally used in pharmaceutical preparations intended for similar purposes.

| Preservatives | % W/V |
|---|---|
| Benzalkonium chloride | 0.004—0.02 |
| Disodium ethylenediaminetetraacetate | 0.01—0.2 |
| Thimerosal | 0.001—0.01 |
| Chlorobutanol | 0.5—1.0 |
| Methyl and/or propyl paraben | 0.01—0.2 |
| Phenethyl alcohol | 0.25—0.75 |
| Cyclohexedine | 0.01—0.1 |

| Viscosity agents | % W/V |
|---|---|
| Methyl cellulose | 0.1—2.0 |
| Hydroxy cellulose | 0.1—2.0 |
| Hydroxypropyl cellulose | 0.1—2.0 |
| Polyvinylpyrrolidone | 0.5—2.0 |

3

In preparing the formulations of the present invention, calcitonin is dissolved in the vehicle or diluent after which the additional ingredients are added in accordance with customary formulation procedures known in the pharmaceutical industry.

Examples of typical intranasal formulations are set forth below.

### Example 1

|  | % W/V |
| --- | --- |
| Calcitonin | 0.009 |
| Sodium Taurocholate | 0.5 |
| Gelatin | 1.0 |
| Purified water Q.S. | 100 |

### Example 2

|  | % W/V |
| --- | --- |
| Calcitonin | 0.009 |
| Sodium Taurocholate | 0.5 |
| Sodium acetate .3H$_2$O | 1.36 |
| Acetic acid | 0.6 |
| Benzalkonium chloride | 0.01 |
| Disodium ethylenediaminetetraacetate | 0.1 |
| Purified water Q.S. | 100 |

### Example 3

|  | % W/V |
| --- | --- |
| Calcitonin | 0.009 |
| Sodium Taurocholate | 0.5 |
| Sodium acetate .3H$_2$O | 1.36 |
| Acetic acid | 1.36 |
| Chlorobutanol | 0.1 |
| Phenethyl alcohol | 0.2 |
| Purified water Q.S. | 100 |

The gelatin used in the above formulations is a standard hydrolipid animal gelatin prepared for pharmaceutical use and routinely used as a diluent for peptides.

According to the present invention, it has been found that calcitonin can be administered intranasally from a vehicle containing absorption promoters with results considerably superior to those obtained with the administration of calcitonin without absorption promoters. The following studies were undertaken to examine the bioavailability of calcitonin from the formulations of the present invention, dependency of intranasal absorption of calcitonin on the level of absorption promoters and stability of calcitonin in the presence of absorption promoters.

Protocol

Male rats weighing 150—250 g were weighed and anesthetized with sodium pentobarbital, 50/mg/kg. by intraperitoneal injection. Once anesthetized the nasopalatine process was occluded with glue. The animals were randomly placed into groups of 5—7 rats with the number of groups being dependent upon the number of intranasal formulations to be tested. Supplemental pentobarbital anesthesia was administered as necessary throughout the study.

Prior to administration of the test material blood was collected by cardiac puncture using a 25G 5/8″ (1.59 cm) needle. Fifty (50) microliters of the salmon calcitonin-containing surfactant solution was then instilled into the nasal septum using polyethylene tubing (PE 20, Peterson, Technics, Monmouth Junction, N.J.) connected to a 1 ml syringe; the tubing was inserted about 1 cm into the nasal septum. One and three hours after nasal installation, blood was again collected by cardiac puncture.

Biochemical analysis

Blood samples were allowed to clot at room temperature and were then refrigerated for 30—60 minutes to provide maximum clot retraction. The samples were centrifuged at 4°C., 5000 rpm for 10 minutes (Beckman Model J2-21 Centrifuge, Beckman Instruments, Palo Alto, CA). Serum calcium was quantitated using a Calcette (Model 4008, Precision Systems, Sudbury, MA).

Data analysis

Serum calcium values at 0, 1 and 3 hours were expressed as mean ± standard deviation. In addition, the absolute change and the percent change from the pretreatment (0 time) value at 1 and 3 hours was also calculated. Statistical analysis consisted of comparison of the serum calcium values at 0 and 1 hour, 0 and 3 hours, and 1 and 3 hours using a t test.

Example 4

This example illustrates decrease in serum calcium in blood samples obtained in accordance with the above protocol when: a. calcitonin is administered alone; b. calcitonin is administered in formulations containing a bile acid salt surface active agent; and c. no calcitonin is present in the formulations.

Table I shows the result obtained.

TABLE I

| Calcitonin U/kg body weight vehicle/surfactant | 0 hour mg/dl | 1 hour | | 3 hour | |
|---|---|---|---|---|---|
| | | mg/dl | % decr. | mg/dl | % decrease |
| 2U 1% gel — | 8.8 | 9.5 | None | 9.9 | None |
| 5U 1% gel — | 8.5 | 7.9 | 7.1 | 9.5 | None |
| 10U 1% gel — | 9.2 | 7.6 | 17.4 | 9.7 | None |
| 10U .1M acetate — | 8.8 | 6.3 | 28.4 | 9.0 | None |
| — 1% gel 1% Taurocholate | 9.1 | 9.5 | None | 9.8 | None |
| 3U 1% gel Taurocholate | 9.1 | 7.0 | 23.1 | 6.4 | 29.6 |
| 10U 1% gel 1% Taurocholate | 9.3 | 7.1 | 23.7 | 6.3 | 32.3 |
| | 8.6 | 6.1 | 29.1 | 5.9 | 31.4 |
| 10U 0.1M acet. 1% Taurocholate | 9.1 | 6.5 | 28.6 | 5.7 | 37.4 |

Example 5

This example illustrates that the enhancement of intranasal absorption depends on the level of absorption promoter present in the formulation.

Table II shows the result obtained.

TABLE II

| 10U Calcitonin/kilo in 0.1M acetate with | | 0 hour mg/dl | 1 hour | | 3 hours | |
|---|---|---|---|---|---|---|
| | | | mg/dl | % | mg/dl | % |
| 1% | Taurocholate | 9.1 | 6.5 | 28.6 | 5.7 | 37.4 |
| 0.5% | „ | 9.0 | 6.1 | 32.2 | 7.5 | 16.6 |
| 0.25% | „ | 9.1 | 6.8 | 25.3 | 7.4 | 18.2 |
| 0.1% | „ | 8.9 | 6.5 | 27.0 | 8.3 | 6.7 |
| 0.05% | „ | 9.0 | 7.6 | 15.5 | 8.7 | 3.3 |

**Claims for the Contracting States: BE, CH, DE, FR, GB, IT, LI, LU, NL, SE**

1. A pharmaceutical composition for the treatment of disorders of bone metabolism which comprises an aqueous or non-aqueous medium suitable for intranasal administration containing a therapeutically effective amount of calcitonin, a bile acid salt surface active agent, and a buffer.

2. The pharmaceutical composition of claim 1 wherein said buffer is from 0.01 to 0.5M.

3. The pharmaceutical composition of claim 1 or 2 further comprising an antioxidant, stabilizer, tonicity adjuster, viscosity builder, or a preservative.

4. The pharmaceutical composition of any of claims 1 to 3 wherein said medium contains from 1 to 150 micrograms calcitonin per ml of said aqueous medium.

5. The pharmaceutical composition of any of claims 1 to 4 containing from 0.01 to 10% w/v of the bile acid salt surface active agent.

6. The pharmaceutical composition of claim 1 wherein said buffer is a phosphate buffer.

7. The pharmaceutical composition of claim 1 wherein said buffer is an acetate buffer.

8. The pharmaceutical composition of any of claims 1 to 7 wherein said aqueous medium is a gel.

9. The pharmaceutical composition of any of claims 1 to 8 wherein said bile acid salt surface active agent is sodium cholate, sodium glycocholate, sodium taurocholate, or sodium deoxycholate.

**Claims for the Contracting State: AT**

1. A process for preparing a pharmaceutical composition for the treatment of disorders of bone metabolism which comprises formulating an aqueous or non-aqueous medium suitable for intranasal administration and a therapeutically effective amount of calcitonin, a bile acid salt surface active agent, and a buffer.

2. The process according to claim 1 wherein said buffer is from 0.01 to 0.5M.

3. The process according to claims 1 or 2 wherein an antioxidant, stabilizer, tonicity adjuster, viscosity builder or a preservative is further added.

4. The process according to any of claims 1 to 3 wherein said medium contains from 1 to 150 micrograms calcitonin per ml of said aqueous medium.

5. The process according to any of claims 1 to 4 wherein the bile acid salt surface active agent is contained in amounts of from about 0.01 to about 10% w/v.

6. The process according to claim 1 wherein said buffer is a phosphate buffer.

7. The process according to claim 1 wherein said buffer is an acetate buffer.

8. The process according to any of claims 1 to 7 wherein said aqueous medium is a gel.

9. The process according to any of claims 1 to 8 wherein said bile acid salt surface active agent is sodium cholate, sodium glycocholate, sodium taurocholate, or sodium deoxycholate.

**Patentansprüche für die Vertragsstaaten BE, CH, DE, FR, GB, IT, LI, LU, NL, SE**

1. Pharmazeutische Zusammensetzung zur Behandlung von Knochenmetaboliestörungen, welche ein wäßriges oder nichtwäßriges Medium, das zur intranasalen Verabreichung geeignet ist, enthaltend eine therapeutisch wirksame Menge Calcitonin, ein Gallensäuresalz als oberflächenaktives Mittel und einen Puffer, umfaßt.

2. Pharmazeutische Zusammensetzung nach Anspruch 1, worin der Puffer 0,01 bis 0,5 M ist.

3. Pharmazeutische Zusammensetzung nach Anspruch 1 oder 2, welche weiterhin ein Antioxidans, einen Stabilisator, ein Tonizitätseinstellmittel, ein Viskositätsaufbaumittel oder ein Konservierungsmittel umfaßt.

4. Pharmazeutische Zusammensetzung nach einem der Ansprüche 1 bis 3, worin das Medium 1 bis 150 Mikrogramm Calcitonin pro ml wäßriges Medium enthält.

**0 115 627**

5. Pharmazeutische Zusammensetzung nach einem der Ansprüche 1 bis 4, welche 0,01 bis 10% w/v (Gewicht/Volumen) des Gallensäuresalzes als oberflächenaktives Mittel enthält.

6. Pharmazeutische Zusammensetzung nach Anspruch 1, worin der Puffer ein Phosphatpuffer ist.

7. Pharmazeutische Zusammensetzung nach Anspruch 1, worin der Puffer ein Acetatpuffer ist.

8. Pharmazeutische Zusammensetzung nach einem der Ansprüche 1 bis 7, worin das wäßrige Medium ein Gel ist.

9. Pharmazeutische Zusammensetzung nach einem der Ansprüche 1 bis 8, worin das Gallensäuresalz als oberflächenaktives Mittel Natriumcholat, Natriumglycocholat, Natrium-taurocholat oder Natrium-deoxycholat ist.

**Patentansprüche für den Vertragsstaat: AT**

1. Verfahren zur Herstellung einer pharmazeutischen Zusammensetzung zur Behandlung von Knochenmetaboliestörungen, welches das Formulieren eines wäßrigen oder nichtwäßrigen Mediums, das zur intranasalen Verabreichung geeignet ist, und einer therapeutisch wirksamen Menge Calcitonin, eines Gallensäuresalzes als oberflächenaktives Mittel und eines Puffers umfaßt.

2. Verfahren nach Anspruch 1, worin der Puffer 0,01 bis 0,5 M ist.

3. Verfahren nach Anspruch 1 oder 2, worin weiterhin ein Antioxidans, ein Stabilisator, ein Tonizitätseinstellmittel, ein Viskositätsaufbaumittel oder ein Konservierungsmittel zugegeben wird.

4. Verfahren nach einem der Ansprüche 1 bis 3, worin das Medium 1 bis 150 Mikrogramm Calcitonin pro ml wäßriges Medium enthält.

5. Verfahren nach einem der Ansprüche 1 bis 4, worin das Gallensäuresalz als oberflächenaktives Mittel in Mengen von etwa 0,01 bis etwa 10% w/v (Gewicht/Volumen) enthalten ist.

6. Verfahren nach Anspruch 1, worin der Puffer ein Phosphatpuffer ist.

7. Verfahren nach Anspruch 1, worin der Puffer ein Acetatpuffer ist.

8. Verfahren nach einem der Ansprüche 1 bis 7, worin das wäßrige Medium ein Gel ist.

9. Verfahren nach einem der Ansprüche 1 bis 8, worin das Gallensäuresalz als oberflächenaktives Mittel Natriumcholat, Natriumglycocholat, Natriumtaurocholat oder Natriumdeoxycholat ist.

**Revendications pour les Etats Contractants: BE, CH, DE, FR, GB, IT, LI, LU, NL, SE**

1. Composition pharmaceutique pour le traitement des désordres du métabolisme osseux, qui comprend un milieu aqueux ou non aqueux approprié pour l'administration intranasale contenant une quantité thérapeutiquement efficace de calcitonine, un sel d'acide biliaire comme agent tensio-actif, et un tampon.

2. Composition pharmaceutique selon la revendication 1, dans laquelle le tampon va de 0,01 à 0,5M.

3. Composition pharmaceutique selon la revendication 1 ou 2, comprenant en outre un agent antioxydant, un agent stabilisant, un agent d'adjustement de la tonicité un adjuvant de viscosité, ou un agent conservateur.

4. Composition pharmaceutique selon l'une des revendications 1 à 3, dans laquelle le milieu contient de 1 à 150 microgrammes de calcitonine par ml dudit milieu aqueux.

5. Composition pharmaceutique selon l'une des revendications 1 à 4, contenant de 0,01 à 10% poids/volume du sel d'acide biliaire comme agent tensio-actif.

6. Composition pharmaceutique selon la revendication 1, dans laquelle le tampon est un tampon phosphate.

7. Composition pharmaceutique selon la revendication 1, dans laquelle le tampon est un tampon acétate.

8. Composition pharmaceutique selon l'une des revendications 1 à 7, dans laquelle le milieu aqueux est un gel.

9. Composition pharmaceutique selon l'une des revendications 1 à 8, dans laquelle le sel d'acide biliaire comme agent tensio-actif est le cholate de sodium, le glycocholate de sodium, le taurocholate de sodium, ou le désoxycholate de sodium.

**Revendications pour l'Etat Contractant: AT**

1. Procédé pour la préparation d'une composition pharmaceutique pour le traitement des désordres du métabolisme osseux, qui consiste à fourmuler un milieu aqueux ou non aqueux approprié pour l'aministration intranasale et une quantité thérapeutique efficace de calcitonine, d'un sel d'acide biliaire comme agent tensio-actif, et un tampon.

2. Procédé selon la revendication 1, dans lequel le tampon va de 0,01 à 0,5M.

3. Procédé selon la revendication 1 ou 2, dans lequel on ajoute encore un agent anti-oxydant, un agent stabilisant, un agent d'adjustement de la tonicité, un adjuvant de viscosité ou un agent conservateur.

4. Procédé selon l'une des revendications 1 à 3, dans lequel le milieu contient de 1 à 150 microgrammes de calcitonine par ml du milieu aqueux.

7

5. Procédé selon l'une des revendications 1 à 4, dans lequel le sel d'acide biliaire comme agent tensio-actif est contenu en des quantités allant d'environ 0,01 à environ 10% poids/volume.

6. Procédé selon la revendication 1, dans lequel le tampon est un tampon phosphate.

7. Procédé selon la revendication 1, dans lequel le tampon est un tampon acétate.

8. Procédé selon l'une des revendications 1 à 7, dans lequel le milieu aqueux est un gel.

9. Procédé selon l'une des revendications 1 à 8, dans lequel le sel d'acide biliaire comme agent tensio-actif est le cholate de sodium, le glycocholate de sodium, le taurocholate de sodium, ou le désoxycholate de sodium.